# EUROPEAN PATENT APPLICATION

(11) **EP 2 153 833 A1**
(43) Date of publication of application: **17.02.2010**
(21) Application number: 08105042.9
(22) Date of filing: 14.08.2008
(51) Int. Cl.: A61K 31/4365, A61P 9/10, A61P 19/02, A61P 29/00

(54) **ADP-receptor antagonist for treatment of inflammatory disease**

(71) Applicant: The Royal College of Surgeons in Ireland, Dublin 2 (IE)
(72) Inventor: The designation of the inventor has not yet been filed
(74) Representative: Purdy, Hugh Barry

(57) **Abstract**

A method of treating cardiovascular complications of inflammatory disease in an individual with an established inflammatory disease and having symptoms of active inflammation comprises a step of treating the individual with a suitable amount of an ADP-receptor antagonist. The inflammatory disease includes a disease selected from the group consisting of rheumatoid arthritis and psoriatic arthritis. The ADP-receptor antagonist may be clopridogel (PLAVIX). The method of the invention has the effect of reducing the risk of a patient with active inflammation having an atherothrombotic event as a side-effect of inflammatory disease. The patients are treated with from 5 to 900mg of an ADP-antagonist daily, with an optional loading dose of an ADP-receptor antagonist of between 300 and 900mg.

## Description

### Introduction

The invention relates to a method of treatment/prevention/management of cardiovascular complications of inflammatory disease in an individual having active inflammatory disease.

Cardiovascular events such as a myocardial infarction (heart attack) and stroke result from thrombotic occlusion of an artery. Platelets play a vital role in the pathogenesis of such a thrombus. Platelet reactivity appears to be a marker for thrombotic risk with recent evidence suggesting that elevated platelet reactivity correlates with elevated thrombotic risk. Elevated thrombotic risk leads to higher cardiovascular risk. Patients with inflammatory arthritis (IA) such as rheumatoid arthritis have a much higher risk of experiencing a cardiovascular event than the normal population. The reasons for this are unclear. Currently, the treatment for cardiovascular complications of inflammatory disease such as inflammatory arthritis is aspirin therapy. This may be a problem for patients with IA because these individuals tend to be taking anti-inflammatory medications which may reduce the effectiveness of aspirin as well as increase their chances of serious bleeding events.

It is an object of the invention to overcome at least one of the above-referenced problems.

### Statements of Invention

The invention is based on the surprising finding that platelets isolated from patients with active inflammatory disease (i.e. patients with inflammatory disease showing symptoms of active inflammation) have elevated reactivity to the platelet agonist ADP compared to patients with non-active inflammatory disease (i.e. patients with inflammatory disease showing no symptoms of active inflammation), and that platelet reactivity to other platelet agonists is not elevated in either active inflammatory disease patients and non-active inflammatory disease patients.

Accordingly, in a first aspect, the invention relates to a method of treating cardiovascular complications of inflammatory disease in an individual with an inflammatory disease, the method comprising a step of treating the individual with a suitable amount of an ADP-receptor antagonist. In particular, the invention relates to a method of treating cardiovascular complications of inflammatory disease in an individual with active inflammation.

The invention also relates to the use of an ADP-receptor antagonist in the treatment of cardiovascular complications of inflammatory disease in an individual with active inflammation.

The invention also relates to a combination therapy for treatment of cardiovascular complications of inflammatory disease in an individual with active inflammation, the combination therapy comprising aspirin and an ADP-receptor antagonist.

The invention also relates to a method of determining a suitable treatment for cardiovascular complications of inflammatory disease in a patient with inflammatory disease, the method comprising a step of measuring the reactivity of the patients platelets to an ADP-receptor agonist, wherein a measured reactivity above a threshold level indicates an ADP-receptor agonist as a suitable treatment, and a measured reactivity at or below the threshold level indicates an alternative treatment.

The invention also relates to a method of treating inflammatory disease in an individual with established inflammatory disease and active inflammation, the method comprising a step of treating the individual with a suitable amount of an ADP-receptor antagonist.

The invention also relates to the use of an ADP-receptor antagonist in the treatment of inflammatory disease in an individual with active inflammation.

The invention also relates to a combination therapy for treatment of an inflammatory disease in an individual with active inflammation, the combination therapy comprising aspirin and an ADP-receptor antagonist.

The invention also relates to a method of determining a suitable treatment for inflammatory disease in a patient with established inflammatory disease, the method comprising a step of measuring the reactivity of the patients platelets to an ADP-receptor agonist, wherein a measured reactivity above a threshold level indicates an ADP-receptor agonist as a suitable treatment, and a measured reactivity at or below the threshold level indicates an alternative treatment.

In this specification, the term "inflammatory disease" should be taken to mean an inflammatory arthriditis selected from the group consisting of Rheumatoid arthritis, Psoriatic arthritis, Ankylosing Spondylitis, Seronegative Spondyloarthropathy,Reactive,Gouty, Juvenile Idiopathic and Stills Disease. In one embodiment of the invention, the term is restricted to rheumatoid arthritis and psoriatic arthritis, especially rheumatoid arthritis.

In this specification, the term "individual with active inflammation" should be taken to a patients with an underlying diagnosis of inflammatory disease, especially rheumatoid arthritis, who exhibits symptoms of the disease following the assessment of disease activity based upon at least one of the patient and doctors assessment of symptoms and functional status, including the evaluation of joints and extra-articular manifestations, and/or laboratory bio-markers (such as elevated fibrinogen, C reactive protein, rheumatoid factor, Anti-CCP, IL-6, Serum albumin) and/or radiological tests.

In this specification, the term "treatment" should be taken to mean reducing the risk of the patient having an atherothrombotic event (such as a myocardial infarction or a thrombosis) as a side-effect of inflammatory disease, and includes both prophylactic treatment of individuals with active inflammatory disease, and, optionally, prophylactic treatment of individuals with inflammatory disease that do not show signs of inflammation, for example individuals with confirmed inflammatory disease and who are being successfully treated for inflammation or individuals with confirmed inflammatory disease who for other reasons do not show active signs of inflammation. Treatment generally entails delivery of the ADP-receptor antagonist into the blood stream of the patients, and any suitable means of delivery is envisaged including oral, inhalable, intravenous, intrathecal, and intramuscular delivery.

In this specification, the term "ADP-receptor antagonist" should be taken to mean an agent that inactivates the human platelet G-protein coupled ADP receptor (P2Y₁₂). An example of such an agent is the drug clopidogrel, sold under the trade name PLAVIX. Other ADP-receptor antagonists include Ticlopidine, Prasugrel, AR-C69931MX, Cangrelor, MRS2179, and the active metabolites thereof.

In this specification, the term "suitable amount" means an amount of an ADP-receptor antagonist suitable for effecting a reduction of platelet reactivity in an individual with confirmed active inflammatory disease to a clinically acceptable level. Typically, this would mean an amount of from 5-900 mg administered daily, suitably from 25-250mg administered daily, typically from 50-200mg administered daily, ideally from 75-150mg administered daily, optionally with a loading dose of 300-900 mg.

In this specification, the term "cardiovascular complications of inflammatory disease" should be taken to mean diseases or conditions associated with thrombosis including stroke, atherosclerosis, coronory artery disease, ischemic cerebrovascular disease, peripheral vascular disease, and other atherothrombotic events, that are recognized by cardiologists and rheumatologists as a side-effect of inflammatory disease.

In this specification, the term "ADP-receptor agonist" should be taken to mean an agent that increases ADP-receptor mediated platelet signaling such as, for example, ADP.

Thus, the invention relates to a method of reducing the risk of a patient with inflammatory disease with symptoms of active inflammation having an atherothrombotic event as a side-effect of the inflammatory disease. The method involves treating the patient with an ADP-receptor antagonist such as PLAVIX. The invention particularly relates to a method of reducing the cardiovascular risk profile of a patient with an inflammatory disease such as inflammatory arthritis who has symptoms of active inflammation, which involves treating the patients with an ADP-receptor antagonist such as PLAVIX. Reducing the cardiovascular risk profile means reducing the risk that the patient has an atherothrombotic event as a side effect of inflammatory arthritis. Typically, the prophylactic treatment regime comprises treating the patient with an ADP-receptor antagonist until the symptoms of active inflammation disappear, and then, optionally, treating the patient with an alternative cardiovascular therapy such as aspirin while the symptoms of inflammation are not present.

The prophylactic treatment of the invention is directed to patients with active inflammatory disease and is especially directed to preventing, or reducing the risk of, atherothrombotic events caused as side-effect of inflammatory disease.

### Brief Description of the Figures

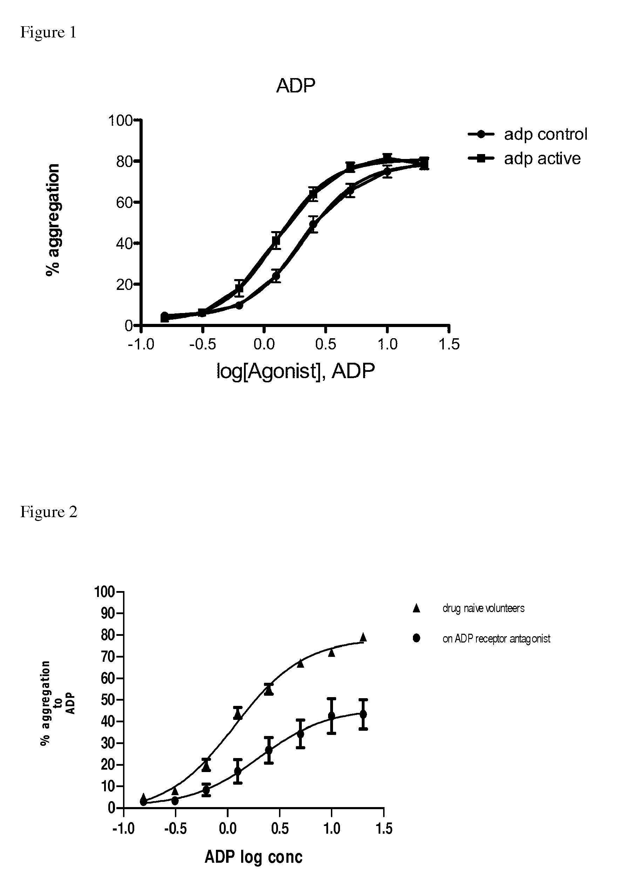

Fig. 1 shows a dose response curve for the agonist ADP generated using conventional serial dilution concentrations of ADP and platelets isolated from patients with active inflammatory arthritis (active ■) and patients with an underlying diagnosis of inflammatory arthritis who at the time of the study did not show signs of inflammation (control ●); and
Fig. 2 shows a dose response curve for the agonist ADP generated using conventional serial dilution concentrations of ADP and platelets isolated from patients undergoing treatment with the ADP-receptor antagonist PLAVIX (●) and drug naive volunteers (▲).

### Detailed Description of the Invention

### Materials and Methods

### Patients

This study was approved by Mater Misericordiae Hospital Medical Research Ethics Committee and complied with the Declaration of Helsinki. Informed consent was obtained from all patients prior to phlebotomy.

### Healthy volunteers

To establish the normal range of platelet response, we tested 80 healthy volunteers (male=40, female=40) who had not taken any medication known to affect platelet function in the previous 14 days. Blood was drawn before 10 AM and 2 hours after a light breakfast to minimise variation due to circadian rhythm. Blood was collected through a 19-gauge Butterfly® needle into a 30-ml syringe containing 3.2% sodium citrate. The first 3ml of blood was discarded. Blood was centrifuged for 10 min at 150g. Platelet-rich plasma (PRP) aspirated from the supernatant was placed in a reagent reservoir. Using a multi-channel pipette, the PRP was dispensed across wells in a 96-well plate (black isoplate ® with clear flat-bottomed wells, PerkinElmer) containing different concentrations of the agonists, arachidonic acid, collagen (type 1 soluble calf skin), Adenosine Diphosphate (ADP), epinephrine and Thrombin Receptor Activating Peptide (TRAP).

### Patients on ADP receptor Antagonists

To establish the normal response to ADP receptor antagonism, we recruited cardiovascular disease patients taking only clopidogrel. All patients were taking chronic clopidogrel in that they had been on clopidogrel for more than 4 weeks.

### Patients with Inflammatory Arthritis

Patients with inflammatory arthritis were recruited from the rheumatology outpatient clinic. All patients with previously documented evidence of inflammatory arthritis were screened for eligibility.
Patients taking aspirin, ADP receptor antagonists, GPIIb/IIIa inhibitors, warfarin, non-steroidal anti-inflammatory medication or any other medication known to affect platelet function were excluded. Patients with a serum creatinine > 150 mmol/l, or a platelet count of < 100,000 / mm³, or who were pregnant, or had hepatic dysfunction (defined by hepatic enzymes more than twice the upper normal limit) were also excluded. After resting comfortably for at least 10 minutes, patients were phlebotomised and blood collected as described above. Demographic material was recorded on all patients.

### Characterisation of Disease Activity

Inflammation was characterised using both clinical and biochemical assessments (C-Reactive Protein, CCP and ESR). In addition, all patients filled in a questionnaire to provide subjective assessment of disease activity. Patients were divided into 2 groups ( to be completed)

### Novel Platelet Function Assay

To assess platelet function, we used a modification of light transmission aggregometry that we have previously described [15, 16]. In brief, 180 µl of PRP was added to each well of a 96-well plate containing different agonists. Light absorbance was measured at standard times. To characterise maximal platelet aggregation, increasing concentrations of agonists were tested. Platelet aggregation measured as a percentage of absorbance from baseline using a 572 nm filter was assayed at 0, 3, 9, 15 and 18 minutes. Between each of the standardised times, the plate was rotated at 1000 r.p.m. through a 0.1 mm orbit. The agonists used were arachidonic acid, collagen, ADP, epinephrine and TRAP. The final concentrations of the agonists arrayed were (500, 375, 188, 93.8, 46.9, 23.4, 11.8, 5.86) µg/ml for arachidonic acid; (190, 143, 71.3, 35.6, 17.8, 8.9, 4.45, 2.23) µg/ml for collagen; (20, 10, 5, 2.5, 1.25, 0.625, 0.313, 0.156) µM for ADP and TRAP; and (20, 5, 1.25, 0.313, 0.078, 0.0195, 0.00488, 0.00122) µM for epinephrine. The agonist volumes used were 50 µl of arachidonic acid, 50 µl of collagen, 40 µl of ADP, 40 µl of epinephrine and 40 µl of TRAP. The 96-well plate was then read using a Victor 3™ Multilabel plate reader (Perkin Elmer, Wellesley, MA, USA). The time from blood draw until the end of the assay protocol was recorded.

### Statistical analysis

Continuous variables were analysed for a normal distribution using the Kolmogorov-Smimov test (using p value > 0.2 as threshold). Continuous variables following a normal distribution are expressed as a mean value +/- standard deviation. The EC50 for each agonist was compared between the active and control groups and analysed using Fisher's exact test. The nominal level of significance was 5%.

### Results

### Patients with Inflammatory Arthritis

A total of 83 inflammatory arthritis patients were recruited from the outpatient clinic. Group 1 consisted of 46 controls with no evidence of inflammation and Group 2 consisted of 27 active patients with definite evidence for active inflammation.
The EC50 from the active group had significantly higher platelet reactivity in response to ADP than the EC50 from the control group (p<0.0001) (see Figure 1). There was no significant difference in EC50 between the 2 groups in response to arachidonic acid, collagen, epinephrine or TRAP (thrombin receptor activating peptide).

### Patients on ADP Receptor Antagonists

Figure 2 shows that the ADP receptor antagonist (in this case Clopidogrel) significantly decreases the aggregation response of platelets to ADP.

### Treatment

Patients with active inflammatory arthritis are treated with the ADP-receptor antagonist PLAVIX according to the following regime: an initial loading dose of 300-900mg followed by administration of 100mg once daily.

The invention is not limited to the embodiments hereinbefore described which may be varied in construction and detail without departing from the spirit of the invention.

## Claims

1. A method of treating cardiovascular complications of inflammatory disease in an individual with an established inflammatory disease and having symptoms of active inflammation, the method comprising a step of treating the individual with a suitable amount of an ADP-receptor antagonist.

2. A method as claimed in Claim 1 in which the inflammatory disease is selected from the group consisting of rheumatoid arthritis and psoriatic arthritis.

3. A method as claimed in Claim 1 or 2 in which the ADP-receptor antagonist is clopridogel (PLAVIX).

4. A method as claimed in any preceding Claim which is a method of reducing the risk of a patient with active inflammation having an atherothrombotic event as a side-effect of inflammatory disease.

5. A method as claimed in any preceding Claim in which the patients is treated with from 5 to 900mg of an ADP-antagonist daily.

6. A method as claimed in any preceding Claim in which the patients is treated with from 25 to 250mg of an ADP-antagonist daily.

7. A method as claimed in any preceding Claim in which the patient is initially trewated with a loading dose of an ADP-receptor antagonist of between 300 and 900mg.

8. A method as claimed in any preceding Claim in which the cardiovascular complications of inflammatory disease are selected from the group consisting of: stroke; atherosclerosis; coronory artery disease; ischemic cerebrovascular disease; and peripheral vascular disease.

9. Use of an ADP-receptor antagonist in the treatment of cardiovascular complications of inflammatory disease in an individual with an established inflammatory disease and active inflammation.

10. Use as claimed in Claim 9 in which the inflammatory disease is selected from inflammatory arthritis or psoriatic arthritis.

11. Use of an ADP-receptor antagonist is the treatment of inflammatory disease is a patient with active inflammation.

12. Use as claimed in Claim 9, 10 or 11 in which the ADP-receptor antagonist is clopidogrel (PLAVIX).
